# EUROPEAN PATENT APPLICATION

(11) **EP 3 489 963 A1**
(43) Date of publication of application: **29.05.2019**
(21) Application number: 17203891.1
(22) Date of filing: 27.11.2017
(51) Int. Cl.: G16H 30/40, G16H 50/20

(54) **AN APPARATUS AND ASSOCIATED METHODS FOR LIFESTYLE RECOMMENDATION GENERATION**

(71) Applicant: Nokia Technologies Oy, 02610 Espoo (FI)
(72) Inventor: BITAULD, David, Cambridge, CB5 8DR (GB)
(74) Representative: Potter Clarkson

(57) **Abstract**

A toilet apparatus comprising: an optical sensor configured to capture optical information of a user's stool within a toilet bowl; and a transmitter configured to transmit the captured optical information of a user's stool to a remote machine learning analyser; wherein the toilet apparatus is configured to: capture optical information of a user's stool using the optical sensor; associate the captured optical information of the stool with the user associated with the stool; and transmit, using the transmitter, the captured optical information and the user association to the remote machine learning analyser for provision of a generated personalised lifestyle recommendation to the user based on a machine learning analysis of the captured optical information. Also a machine learning analyser configured to: receive captured optical information of a stool associated with a user; and use machine learning analysis to generate a personalised lifestyle recommendation for provision to the user based on the captured optical information.

## Description

### Technical Field

The present disclosure relates to apparatus and methods associated with capturing optical information (e.g. photographic images, spectra) from user's stools and using machine learning to generate personalised lifestyle recommendations from that optical information.

Some examples may relate to portable electronic devices, in particular, so-called hand-portable electronic devices which may be hand-held in use (although they may be placed in a cradle in use). Such hand-portable electronic devices include so-called Personal Digital Assistants (PDAs) and tablet PCs. Certain portable electronic devices may be wearable, such as on the wrist. The portable electronic devices/apparatus according to one or more disclosed example aspects/embodiments may provide one or more audio/text/video communication functions (e.g. telecommunication, videocommunication, and/or text transmission, Short Message Service (SMS)/ Multimedia Message Service (MMS)/emailing functions, interactive/non-interactive viewing functions (e.g. web-browsing, navigation, TV/program viewing functions), music recording/playing functions (e.g. MP3 or other format and/or (FM/AM) radio broadcast recording/playing), downloading/sending of data functions, image capture function (e.g. using a (e.g. in-built) digital camera), and gaming functions.

### Background

Recent developments in technology include devices used to monitor a user's health and help the user improve their diet and/or lifestyle.

The listing or discussion of a prior-published document or any background in this specification should not necessarily be taken as an acknowledgement that the document or background is part of the state of the art or is common general knowledge.

### Summary

According to a first aspect, there is provided a toilet apparatus comprising:
an optical sensor configured to capture optical information of a user's stool within a toilet bowl; and
a transmitter configured to transmit the captured optical information of a user's stool to a remote machine learning analyser;
wherein the toilet apparatus is configured to:
   capture optical information of a user's stool using the optical sensor;
   associate the captured optical information of the stool with the user associated with the stool; and
   transmit, using the transmitter, the captured optical information and the user association to the remote machine learning analyser for provision of a generated personalised lifestyle recommendation to the user based on a machine learning analysis of the captured optical information.

The captured optical information and the user association may be indirectly transmitted from the toilet apparatus to the remote machine learning analyser via a further computing device. The further computing device may be, for example, a mobile phone, a tablet computer, a desktop computer, a laptop computer, a server, a wearable device, a wristband device, a fitness tracker device, a smartwatch, a user device or a third-party device. Transmission may be wired or wireless. Of course, the captured optical information would be in a format for wireless/wired transmission by a computer.

The toilet apparatus may be one of: a toilet bowl, a toilet seat, an attachable module for a toilet bowl and an attachable module for a toilet seat.

The optical sensor may be configured to capture one or more of: a standard camera image, a stereoscopic image, a hyperspectral image, and a spectrometer measurement. Different types of spectrometer image may be captured, such as a microspectrometer image captured using a microspectrometer.

The toilet apparatus may be configured to associate the captured optical information of the stool with the user associated with the stool by receiving a user identifier from the user. The user identifier may be any method by which the user can be associated with the captured optical information of the stool, for example by voiceprint, user name, user ID code, fingerprint, or other identification means.

The toilet apparatus may further comprise an output user interface configured to provide the personalised lifestyle recommendation to the user.

The toilet apparatus may further comprise a receiver configured to receive the generated personalised lifestyle recommendation from the remote machine learning analyser. The receiver and transmitter may be present together as a transceiver. The receiver, transmitter, and/or transceiver may be wired and/or wireless.

According to a further aspect, there is provided a machine learning analyser configured to:
receive captured optical information of a stool associated with a user; and
use machine learning analysis to generate a personalised lifestyle recommendation for provision to the user based on the captured optical information.

The machine learning analyser may be configured to use machine learning analysis by:
analysing the captured optical information of the user's stool with captured further optical information of one or more other stools having respective associated machine learnt lifestyle outcomes, wherein the captured further optical information of the one or more other stools are from members of a population class linked to the user.

The machine learning analyser may be configured to use the machine learning analysis that is trained by associating respective lifestyle outcomes associated with further optical information of stools, wherein the further optical information of stools is from members of a population class linked to the user; and based on the trained machine learning analysis, associate the captured optical information of the user's stool with the optical information of one or more of respective associated machine learnt lifestyle outcomes.

The machine learning analyser may be configured to generate a personalised lifestyle recommendation based on the respective associated machine learnt lifestyle outcomes associated with the captured optical information of the user's stool.

The personalised lifestyle recommendation may be provided to the user by the machine learning analyser. The provision may be made directly to a device of the user, the device of the user being one or more of: a portable electronic device, a mobile phone, a tablet computer, a desktop computer, a laptop computer, a server, a wearable device, a wristband device, a fitness tracker device, a smartwatch.

The further optical information of stools from members of the population class linked to the user may comprise previous captured optical information of stools associated with the user. Thus, the machine learning analyser may take into account both captured optical information of stools from other members of the population class linked to the user, as well as captured optical information of stools from the user.

The machine learnt lifestyle outcomes for the members of the population class linked to the user may be based on one or more captured physiological indicators, the captured physiological indicators indicating one or more of: heart rate, breathing rate, blood pressure, weight, BMI, body temperature, body hydration, body perspiration, stress levels, glucose levels, blood oxygenation; and sleep activity.

The machine learnt lifestyle outcomes for the members of the population class linked to the user may be based on one or more foods consumed by a member of the population class and associated with captured optical information of a stool of that member. For example, the members may keep a food diary to link food intake with stool production.

The population class may comprise at least: 10, 20, 50, 100, 200, 300, 400, 500, 750, 1000, 2000, 5000, or more than 5000 members.

The population class linked to the user may be linked by sharing one or more demographic factors with the user. Example demographic factors include: gender, age, weight, height, ethnicity, occupation, income, geographic location, overall activity level, health factors, special dietary requirements, medical conditions, and user preferences. Each demographic factor may be considered to be shared between a member and the user if the factors for the member and the user are the same within a predefined tolerance for the user.

The machine learning analyser may be further configured to:
identify a value of a characterising parameter of the stool in the captured optical information;
associate the value of the characterising parameter with a machine learnt lifestyle outcome; and
generate the personalised lifestyle recommendation for provision to the user based on the associated value of the characterising parameter.

The characterising parameter may be: stool volume; stool colour profile; the presence of one or more predetermined colours; stool surface texture; stool shape; and stool fragmentation.

The lifestyle outcomes may indicate one or more of: a characteristic of the user's stool; a diagnosis of a medical condition; an activity recommendation and a dietary recommendation. The personalised lifestyle recommendation may also provide one or more of these indications.

An activity recommendation may comprise one or more physical activities the user should avoid, and/or one or more physical activities the user should perform.

A dietary recommendation may comprise one or more foods or food groups the user should avoid, one or more foods or food groups the user should consume, a recipe idea comprising one or more foods the user should consume, and/or a recipe idea omitting one or more foods the user should avoid.

A characteristic of the user's stool may comprise one or more of: stool volume; stool colour profile (e.g. the overall colour palette); the presence of one or more predetermined colours (e.g. dark red, white); stool surface texture (e.g. smooth, cracked, fluffy); stool shape (e.g. thin, fat); and stool fragmentation (e.g. several small pieces, one single piece, liquid).

The machine learning analyser may be further configured to provide the generated personalised lifestyle recommendation by transmission to a user output device of one or more of:
a visual message to display the generated personalised lifestyle recommendation,
an audio message to provide audio output indicating the generated personalised lifestyle recommendation, and
a haptic feedback indication to prompt the user that a personalised lifestyle recommendation has been generated.

The further captured optical information of one or more other stools from members of a population class linked to the user may comprise one or more of:
an image captured using the optical sensor of toilet apparatus of claim 1;
an image captured using an optical sensor which is not part of the toilet apparatus of claim 1;
a schematic representation; and
a computer simulation image reconstructed from a description.

In a further aspect there is provided a computer-implemented method comprising:
capturing optical information of a user's stool;
associating the captured optical information of the stool with the user associated with the stool; and
transmitting the captured optical information and the user association to a remote machine learning analyser for provision of a generated personalised lifestyle recommendation to the user based on a machine learning analysis of the captured optical information.

In a further aspect there is provided a computer-implemented method comprising:
receiving captured optical information of a stool associated with a user;
analysing the captured optical information based on a machine learning analysis to generate a personalised lifestyle recommendation for provision to the user; and
outputting the generated personalised lifestyle recommendation for provision to the user.

The steps of any method disclosed herein do not have to be performed in the exact order disclosed, unless explicitly stated or understood by the skilled person.

In a further aspect there is provided a system comprising a toilet apparatus and a machine learning analyser;
the toilet apparatus comprising:
   an optical sensor configured to capture optical information of a user's stool within a toilet bowl; and
   a transmitter configured to transmit the captured optical information of a user's stool to a remote machine learning analyser;
   wherein the toilet apparatus is configured to:
      capture optical information of a user's stool using the optical sensor;
      associate the captured optical information of the stool with the user associated with the stool; and
      transmit, using the transmitter, the captured optical information and
   the user association to the remote machine learning analyser; and
the machine learning analyser configured to:
   receive the captured optical information of the stool and the user association; and
   analyse the captured optical information based on a machine learning analysis to generate a personalised lifestyle recommendation for provision to the user.

Corresponding computer programs for implementing one or more steps of the methods disclosed herein are also within the present disclosure and are encompassed by one or more of the described examples.

In a further aspect there is provided a computer readable medium comprising computer program code stored thereon, the computer readable medium and computer program code being configured to, when run on at least one processor, perform the method of:
capturing optical information of a user's stool;
associating the captured optical information of the stool with the user associated with the stool; and
transmitting the captured optical information and the user association to a remote machine learning analyser for provision of a generated personalised lifestyle recommendation to the user based on a machine learning analysis of the captured optical information.

In a further aspect there is provided a computer readable medium comprising computer program code stored thereon, the computer readable medium and computer program code being configured to, when run on at least one processor, perform the method of:
receiving captured optical information of a stool associated with a user;
analysing the captured optical information based on a machine learning analysis to generate a personalised lifestyle recommendation for provision to the user; and
outputting the generated personalised lifestyle recommendation for provision to the user.

One or more of the computer programs may, when run on a computer, cause the computer to configure any apparatus, including a circuit, controller, or device disclosed herein, or perform any method disclosed herein. One or more of the computer programs may be software implementations, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

One or more of the computer programs may be provided on a computer readable medium, which may be a physical computer readable medium such as a disc or a memory device, may be a non-transient medium, or may be embodied as a transient signal. Such a transient signal may be a network download, including an internet download.

The present disclosure includes one or more corresponding aspects, examples or features in isolation or in various combinations whether or not specifically stated (including claimed) in that combination or in isolation. Corresponding means (e.g. optical information-stool associator, captured optical information receiver, lifestyle recommendation generator) for performing one or more of the discussed functions are also within the present disclosure.

The above summary is intended to be merely exemplary and non-limiting.

### Brief Description of the Figures

A description is now given, by way of example only, with reference to the accompanying drawings, in which:
Figure 1a shows an example toilet apparatus according to the present disclosure;
Figure 1b shows an example apparatus according to the present disclosure;
Figure 2 shows an example toilet apparatus and machine learning analyser apparatus according to the present disclosure;
Figure 3 shows an example of training a machine learning analyser according to the present disclosure;
Figure 4 shows an example of using a machine learning analyser according to the present disclosure;
Figures 5a and 5b shows the main steps of methods of using the present apparatus; and
Figure 6 shows a computer-readable medium comprising a computer program configured to perform, control or enable the methods of Figures 5a and 5b.

### Description of Specific Examples

Recent developments in technology include devices used to monitor a user's health and help the user improve their diet and/or lifestyle.

Since the beginnings of medicine, doctors have used the appearance of stools (faeces) to evaluate the health of their patient. So-called Bristol charts have been used to correlate stool appearance with medical conditions. Stools can be a rich indicator about general health and, in combination with, for example, food logging and other data available from devices connected to the user, such as wearable fitness/lifestyle devices (e.g. blood pressure monitor, heart rate monitor, pedometer), it is possible to obtain a complex assessment of a user's general health, identify problems and suggest behaviour changes. Inspection of stools may be especially important for people suffering from food allergies or tolerances. If the process is made easy to use, it could be beneficial for people who don't have allergies but want to improve their diet and/or lifestyle.

Determining what type of diet makes someone feel better is a difficult process, because it is tedious to keep track of food intake and difficult to quantify how we feel as a result. Keeping a log of stool appearance (as can be required for people suffering from Crohn's Disease, other digestive illnesses, or other food sensitivities and allergies) is even more tedious. Also, the human eyes and brain have limitations for assessing stools, such as limited colour perception, imprecise volume estimation, and emotion-dependent judgement.

It may be useful, and more hygienic, to be able to extract useful information from a user's stools without requiring direct contact and physical sampling of the stool. One potential stool analysis method to capture information from a user's stool may be remote imaging (such as optical, volumetric and spectral imaging).

By using information obtained from a user's stool, it may be possible to provide the user with a suggested behaviour change to improve the user's health, as understood from the information obtained from the user's stool. As a simple example, if a user's stool is formed of several small distinct pieces, this may indicate constipation and the user's health may be improved if they drank more water, ate more dietary fibre, ate a lower fat and sugar diet, and/or performed more exercise, for example.

One or more examples disclosed herein may provide the suggested uses and improvements mentioned above.

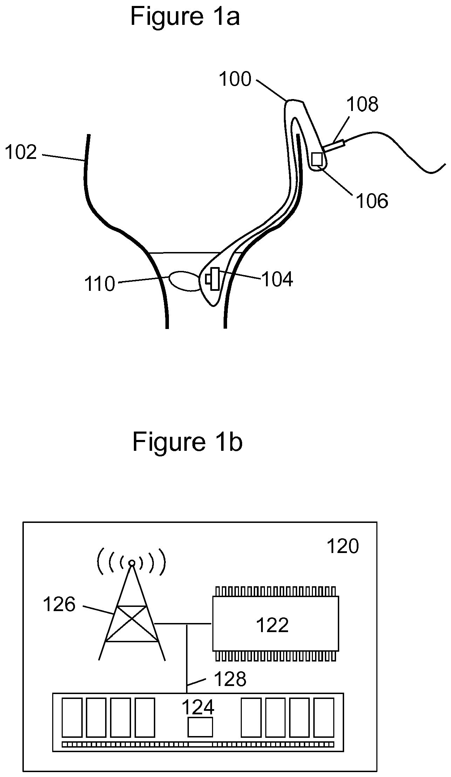

Figure 1 a shows an example of a toilet apparatus 100. The toilet apparatus 100 comprises an optical sensor 104 which is configured to capture optical information of a user's stool 110 within a toilet bowl 102. The optical sensor 104 may be a standard camera configured to take a photographic image, and/or may be a non-standard camera able to take a non-standard optical image. For example, the optical sensor 104 may be configured to capture a standard camera image, a stereoscopic image, a hyperspectral image, and/or a spectrometer measurement (such as a microspectrometer image). Different types of image may provide different information about the user's stool. Optical sensors such as stereoscopic/3D imaging sensors, hyperspectral imaging sensors, and micro-spectrometers, can be low cost and can be compact enough for this application of being part of a toilet apparatus as claimed. In some examples there may be more than one optical sensor. For example, there may be different optical sensors configured to capture different image information. There may be more than one of the same type of optical sensor located at different positions within the toilet base, e.g. to image different portions of the user's stool.

In some examples, the terms "image" and "optical information" used herein may refer to a photographic-like representation of the user's stool (such as a 2D photograph, or a volumetric/3D image). In some examples, spectral information (such as a colour distribution or intensity spectrum) may be obtained from the photographic-like representation, and this spectral information may be termed an "image" or "optical information". In some examples, the terms "image" and "optical information" may refer to spectral information directly obtained from the user's stool by the optical sensor, without a photographic-like representation being captured. In some examples both a photographic-like representation and spectral information may be obtained by one or more optical sensors.

The toilet apparatus 100 in this example also comprises a wireless transmitter 106 which is configured to wirelessly transmit the captured optical information of a user's stool to a remote machine learning analyser. In this example transmission is made over a wireless connection, but in other examples transmission may be made over a wired connection using a wired transmitter.

The toilet apparatus 100 as a whole is configured to capture optical information of a user's stool 110 using the optical sensor 104, associate the captured optical information of the stool with the user associated with the stool 110; and transmit, using the transmitter 106, the captured optical information and the user association to the remote machine learning analyser for provision of a generated personalised lifestyle recommendation to the user based on a machine learning analysis of the captured optical information.

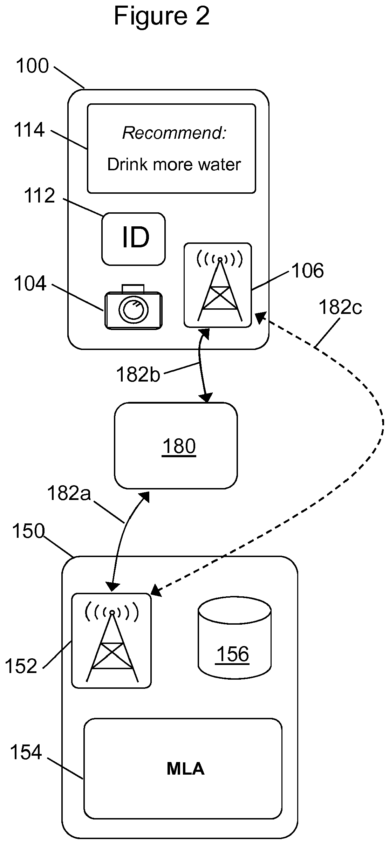

The user association indicates which person produced the imaged stool. For example, several different people may all share the same toilet in a household. By associating a particular user with the produced and imaged stool, personalised lifestyle recommendations can be generated for that particular user. Identifying a user, to be able to associate a particular user with a particular imaged stool, are discussed in relation to Figure 2.

The toilet apparatus 100 shown in Figure 1 a is an attachable module for a toilet bowl, which may hook over the side of the toilet bowl and be removable (for example, for cleaning or maintenance). The toilet apparatus 100, and more specifically the optical sensor 104 and transmitter 106 of the toilet apparatus 100, may be powered by a connection to the mains electricity 108, a connection to an external battery 108, by an internal replaceable or rechargeable battery, or otherwise (e.g. by solar power). In other examples, the toilet apparatus 100 may be an attachable module for a toilet seat (e.g. it may hook onto, or otherwise attach to, a toilet seat). In other examples, the toilet apparatus 100 may be integrated into the toilet and so may be the toilet seat or the toilet bowl.

Figure 1b shows an apparatus 120 which may form part of the toilet seat apparatus 100 of Figure 1a. The apparatus 120 comprises a processor 122 and memory 124 (including computer program code) and a transceiver 126, which are electrically connected to one another by a data bus 128.

The processor 122 may be configured for general operation of the apparatus 120 by providing signalling to, and receiving signalling from, the other components to manage their operation. The memory 124 may be configured to store computer code configured to perform, control or enable operation of the apparatus 120. The memory 124 may also be configured to store settings for the other components (including an optical sensor 104 shown in Figure 1a, and a user identification apparatus (not shown)). The processor 122 may access the memory 124 to retrieve the component settings in order to manage the operation of the other components.

The transmitter 126 may comprise a separate transmitter and receiver and is configured to transmit data to, and receive data from, one or more other devices, such as a machine learning analyser or a further computer apparatus in communication with a machine learning analyser, via a wireless or a wired connection. For example, if the apparatus 120 forms part of a toilet apparatus 100, the transceiver 126 may be configured to receive the captured optical information of the user's stool to a machine learning analyser, and/or provide a personalised lifestyle recommendation for provision to the user.

Figure 2 illustrates schematically a system comprising a toilet apparatus 100 and a machine learning analyser 150. The toilet apparatus 100, as in Figure 1a, comprises an optical sensor 104, a transmitter 106, as well as a user identification apparatus 112, and a user output interface 114. In other examples the toilet apparatus may not comprise a user identification apparatus 112 or a user output interface 114, but may be in communication (wired or wireless) with an external user identification apparatus and a user output interface. The external user output interface may be comprised in a user device, such as a smartphone, smartwatch, or other personal electronic device.

The machine learning analyser 150 is configured to receive (e.g. at receiver/transceiver 152) captured optical information of a stool associated with a user; and configured to use machine learning analysis (e.g. using a machine learning analyser module 154) to generate a personalised lifestyle recommendation for provision to the user based on the captured optical information.

In some examples the machine learning analyser 150 may be a remote computing device such as a server, desktop computer or personal electronic device. The machine learning analyser 150 may be present in a local area to the toilet apparatus 100 and may communicate e.g. over a WLAN. In some examples the machine learning analyser 150 may be remote in a different geographic location to the toilet apparatus 100, and may communicate, for example, over the Internet.

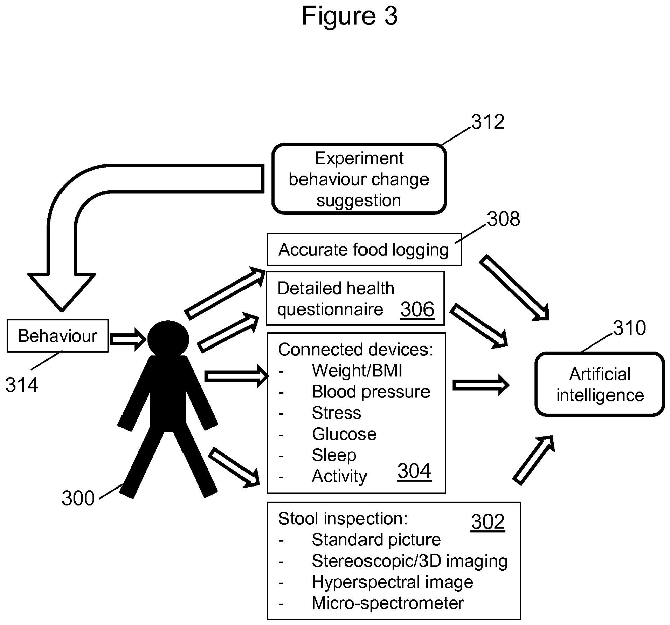

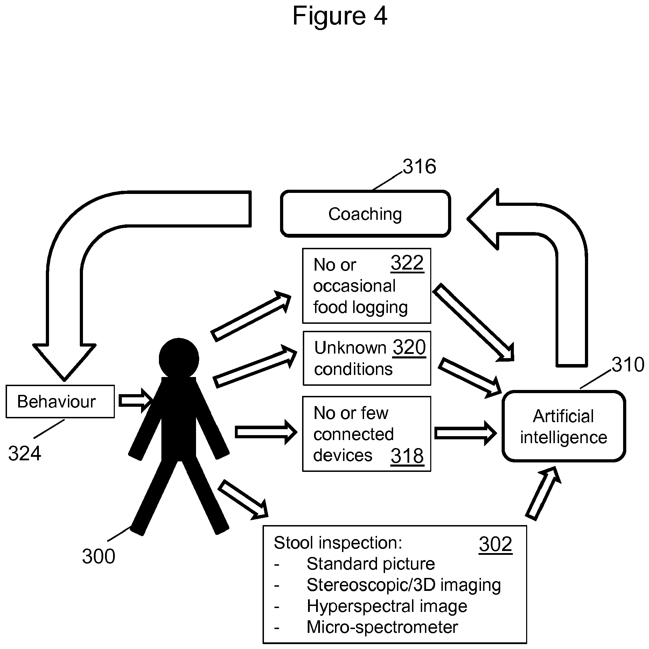

The machine learning analyser 150 in this example comprises a transceiver 152 for communication with the toilet apparatus 100, a machine learning analysis module 154 used for analysis of the captured optical information of the user's stool, and a storage medium 156 (e.g. a database) for the storage of one or more of previous user stool data, and stool data for one or more other users (e.g. training data). The data stored in the database 156 may be used by the machine learning analysis module 154 to generate a personalised lifestyle recommendation for provision to the user. The operation of the machine learning analyser 150 is described in more detail with reference to Figures 3 and 4. In other examples the machine learning analyser 150 may not comprise such a database but may be in communication with such a database.

In some examples the machine learning analysis module 154 may be a virtual module (software) running on an external server or "cloud".

The toilet apparatus 100 is configured to capture optical information of a user's stool using the optical sensor 104; associate the captured optical information of the stool with the user associated with the stool (in this example by using the user identification apparatus 112); and transmit, using the transmitter 106, the captured optical information and the user association to the remote machine learning analyser 150. The machine learning analyser 150 is configured to receive the captured optical information of the stool and the user association (for example using a receiver or transceiver 152); and analyse the captured optical information based on a machine learning analysis 154 to generate a personalised lifestyle recommendation for provision to the user.

The captured optical information and the user association may be indirectly transmitted 182b, 182a from the toilet apparatus 100 to the remote machine learning analyser 150 via a further computing device 180. The further computing device 180 may be, for example, a mobile phone, a tablet computer, a desktop computer, a laptop computer, a server, a wearable device, a wristband device, a fitness tracker device, a smartwatch, a user device or a third-party device. Transmission may be made over two wired connections, over two wireless connections, or over a combination of wired and wireless connections.

The toilet apparatus 100 may be configured to associate the captured optical information of the stool with the user associated with the stool by receiving a user identifier from the user, for example using a user identification apparatus 112. The user identifier may be any method by which the user can be associated with the captured optical information of the stool, for example by voiceprint (e.g. the user recites an expected word or phrase for detection by the user ID apparatus 112 to determine the identity of the user), user name, user ID code (e.g. entered using a keypad or touchscreen), fingerprint, or other identification means. Thus, the user identification apparatus 112 may comprise a microphone, and/or an input user interface such as a keyboard/keypad or touch screen for a user to enter a username or ID code, or a fingerprint scanner. In some examples there may be a fingerprint scanner separate from and in communication with the toilet apparatus 100, such as on a part of the toilet which the user is likely to contact, e.g. a flush handle. In this way the user need not consciously remember to provide user identification when they use the toilet to pass a stool, since they will contact the flush handle anyway and register their fingerprint.

The user output interface 114 of the toilet apparatus 100 may provide a visual message to display the generated personalised lifestyle recommendation. In such examples the user output interface may be a display screen such as an LCD screen, touch sensitive screen, or e-ink screen. In some examples the user output interface 114 may be an audio user output interface 114 which may provide audio message output indicating the generated personalised lifestyle recommendation as an announcement, and/or may provide an audible indication such as a beep that a recommendation is available for the user. A haptic feedback user output interface 144 may indicate to the user that a personalised lifestyle recommendation has been generated (e.g. a vibration may indicate that a recommendation is available for the user). In other examples the user output interface 114 may provide a signal, such as a beep or vibration, to indicate a personalised lifestyle recommendation has been provided to a user device, such as a mobile phone or smartwatch, for output the user.

The generated personalised lifestyle recommendation may be provided to the toilet apparatus 100 from the machine learning analyser 150, either directly 182c, or via a further computing device 180; 182a, 182b. In some examples the user may receive the generated personalised lifestyle recommendation via a user output interface which is not part of the toilet apparatus 100, such as a user interface of the user's personal mobile telephone, wearable fitness apparatus, smartwatch, or other personal computing device.

The toilet apparatus 100 may comprise a receiver (which may be a transceiver 106) configured to receive the generated personalised lifestyle recommendation from the remote machine learning analyser 150. In both the toilet apparatus 100 and the machine learning analyser 150, the receiver and transmitter may be present together as a transceiver 106, 152.

The operation of the machine learning analyser 150 is described with reference to Figures 3 and 4. The machine learning analyser 150 in general uses artificial intelligence (AI) to suggest behaviour (e.g. diet, activity, medication) changes for a user based on stool data for the user captured using a toilet apparatus.

Figure 3 illustrates the training phase of the machine learning analyser (also known as the "infer" or "inference" phase). In a training phase, a machine learning analyser (artificial intelligence) 310, learns the correlations between as many data sources as possible. The data sources may be stool images 302 and health/user data 304, 306, 308 from other users 300. The other users 300 (who are not the end user) may be considered to be members of the population class which is linked to the user. The population class may comprise at least: 10, 20, 50, 100, 200, 300, 400, 500, 750, 1000, 2000, 5000, or more than 5000 members. It may be that more reliable personalised lifestyle recommendations may be provided for a user if there is a larger pool of training data used in the training phase, for example to reduce the influence of statistical anomalies in the training data.

Examples of machine learning algorithms which may be used include neural networks, deep learning, Bayesian learning, reinforcement learning, genetic algorithms, and rule-based machine learning.

The users 300 may or may not comprise the end user receiving the personalised lifestyle recommendations based on the captured optical information of their stool when the machine learning analyser is operating in the "use" phase (see Figure 4). In some examples, the data used in the training phase to train the machine learning analyser may comprise captured optical information of stools from members of a population class linked to the user, but not the user himself. In some examples, when considering the training data which is used by the machine learning analyser to predict a personalised lifestyle recommendation for a user, captured optical information of stools 302 from members of the population class linked to the user which are provided in the training phase may comprise previous captured optical information of one or more stools associated with the user. Thus, the machine learning analyser may take into account both captured optical information of stools from other members of the population class linked to the user, as well as captured optical information of stools from the user.

The population class linked to the user may be linked by sharing one or more demographic factors with the user. Example demographic factors include: gender, age, weight, height, ethnicity, occupation, income, geographic location, overall activity level, health factors, special dietary requirements, medical conditions, and user preferences. For example, it may be that stool appearance correlates with physical activity levels differently for men than for women (e.g. increasing physical activity may have a strong benefit to improve poor digestion in women but only a weak benefit to improve poor digestion in men). In such a case, only data from women users recorded in the training phase may be used if the end user is a woman.

Each demographic factor may be considered to be shared between a member and the user if the factors for the member and the user are the same within a predefined tolerance for the user. For example, if a user is age 37, members of a population class linked to the user may be aged in the age range of 35 - 39 (i.e. ± 2 years compared to the user). Other predefined tolerances may be used.

It will be appreciated that the selection of training data as being applicable in the use phase for a particular user may be dependent on the user's stool appearance (and on other factors if they are recorded and provided to the machine learning analyser with the captured optical information of the user's stool, such as the user's recent diet, activity levels, and any current medical condition). Therefore, the machine learning algorithm can adjust which training data is considered to be relevant based on one or more factors of the current user including the defining features of the captured optical information of the user's stool as captured using the optical sensor, in order to provide relevant personalised lifestyle recommendations for the user's current condition.

The stool images 302 of the users taken in the training phase may be any captured optical information from members of a population class linked to the user (which may or may not include the user), such as a standard camera image, a stereoscopic image/3D image, a hyperspectral image, and a spectrometer measurement. The captured further optical information of one or more other stools 302 may comprise, for example, an image captured using the optical sensor of a toilet apparatus configured to communicate with the machine learning analyser (for example, the toilet apparatus of the user himself or someone in the user's household); an image captured using an optical sensor which is not part of the toilet apparatus of claim 1 (e.g. in a separate data capture in another household using a toilet apparatus as described here, or by an image capture device under laboratory conditions); a schematic representation (e.g. a medical or technical representation); and/or a computer simulation image reconstructed from a description.

Data provided from connected devices 304 in the training phase may be any type of health-related information provided by the users 300 providing stool images. For example, users 300 may undergo one or more forms of health/physiological monitoring during provision of the stool image data in the training phase so that the machine learning analyser can correlate the stool images with the corresponding physiological data for the user. In other words, the machine learnt lifestyle outcomes for the members of the population class linked to the user 300 may be based on one or more captured physiological indicators 304. The captured physiological indicators may indicate one or more of: heart rate, breathing rate, blood pressure, weight, BMI, body temperature, body hydration, body perspiration, stress levels, glucose levels, blood oxygenation; and sleep activity. Such physiological indicators may be recorded using a wearable device. For example, if a member 300 produces a stool of a particular appearance, and the member is monitored using a blood pressure monitor and determined to have high blood pressure at the time of stool production, then it may be inferred in the use phase that another end-user producing a stool of similar appearance may be predicted to have high blood pressure.

Data may also be provided by the members 300 in the training phase in the form of a health questionnaire 306 detailing the member's medical history, family medical history, symptoms, allergies, food intolerances, current psychological state, and current medication, for example. In other words, the machine learnt lifestyle outcomes for the members of the population class linked to the user 300 may be based on one or more medical/health factors of the member of the population class and associated with captured optical information of a stool of that member 300. For example, if a member 300 produces a stool of a particular appearance, and the member has recorded that they suffer from Crohn's disease, then it may be inferred that another user producing a stool of similar appearance may also have undiagnosed Crohn's disease and needs to see a medical professional.

Data may also be provided by the members 300 in the training phase in the form of a diet log 308 detailing what the user has eaten, when, and in what quantities, for example. In this way, the members 300 can record a food diary to link food intake with stool production. In other words, the machine learnt lifestyle outcomes for the members of the population class linked to the user 300 may be based on one or more foods consumed 308 by a member of the population class and associated with captured optical information of a stool of that member 300. For example, if a member 300 produces a stool of a particular appearance, and the user is monitored and determined to have eaten a high fat diet just prior to the time of stool production, then it may be inferred that another user producing a stool of similar appearance has also been eating a high fat diet. A personalised lifestyle recommendation may then be provided to that user, for example indicating that a lower fat diet would be a healthier choice.

Of course, the above examples are very simplified and the machine learning algorithm 310 can take into account many different combinations of demographic factors, heath and dietary indications to provide the user with a personalised lifestyle recommendation. In general, it may be said that the machine learning analyser is configured to use machine learning analysis by: analysing the captured optical information of the user's stool with captured optical information of one or more other stools having respective associated machine learnt lifestyle outcomes, wherein the captured optical information of one or more other stools are from members of a population class linked to the user.

By analysing the captured optical information of the user's stool using a machine learning analyser/program which has been trained (and may be considered "optimised") using the captured optical information of one or more other stools from members of a population class linked to the user, the machine learning analyser may determine a lifestyle recommendation for the user based on the machine learnt lifestyle outcomes for the one or more other users in the population class linked to the user.

The machine learning analyser may be configured to use the machine learning analysis that is trained by associating respective lifestyle outcomes associated with further optical information of stools, wherein the further optical information of stools is from members of a population class linked to the user; and based on the trained machine learning analysis, associate the captured optical information of the user's stool with one or more of the respective associated machine learnt lifestyle outcomes.

The machine learning analyser may, in some examples, be configured to analyse the captured optical information of the user's stool by comparing the captured optical information of the user's stool with the captured further optical information of one or more stools from members of the population class linked to the user; and matching the captured optical information of the user's stool with matching captured optical information of one or more other stools. The comparing and matching steps may not necessarily be direct comparison of one image from the user with another from a comparable user, but may be a machine learning process of determining one or more matching factors or aspects of the captured images to provide a meaningful lifestyle recommendation based on the machine learnt "knowledge" the machine learning analyser has from being trained with the previous images from the one or more other users.

The "machine learnt lifestyle outcomes" are known lifestyle factors of the users sampled in the training phase and linked to images of particular stools for that user. For example, a stool produced by an obese man with Coeliac's disease has a machine learnt lifestyle outcome of "diagnosed with Coeliac's disease" and "would benefit from losing weight due to obesity". The lifestyle outcomes are the known lifestyle factors for the members whose data is captured in the training phase of the machine learning analyser 150.

Further, in general, it may be said that the machine learning analyser 310 is configured to generate a personalised lifestyle recommendation by based on the respective associated machine learnt lifestyle outcomes associated with the captured optical information of the user's stool. This may in some examples be achieved by identifying the respective associated machine learnt lifestyle outcomes of the captured optical information of one or more matching stools; and using the identified respective associated machine learnt lifestyle outcomes to determine the personalised lifestyle recommendation to the user.

Training the machine learning analyser 310 and using it to analyse a user's stools may highlight correlations between the type of food ingested and the resulting stools, which in turn can provide a comprehensive indicator of physical and even psychological wellbeing.

In some examples, behaviour changes 314 may be tested on users/patients in continued experiments to determine the outcome of known lifestyle changes under known conditions. In this case the training phase would be a supervised training phase of the machine learning analyser.

Once the training phase is completed the machine learning analyser may be considered to be "optimised" and ready to analyse an end-user stool. Figure 4 illustrates the "use" phase of the machine learning analyser 310 following training. In a use phase, the machine learning analyser (artificial intelligence) 310, such as a neural network, uses the data provided in the training phase to predict an outcome for the user. The captured optical information of the user's stool 302 is provided to the trained machine learning analyser 310 ("artificial intelligence") to obtain a personalised lifestyle recommendation 316 ("coaching"). The user may follow the personalised lifestyle recommendation 316 and change their behaviour 324 which may influence future stool appearance 302.

In some examples, the user 300 may also provide some additional data 318, 320, 322 (but this additional data would likely be much less than is provided in the training phase). The additional data may comprise: health indications 318 from one or more devices connected to the user, such as blood pressure or heart rate; an occasional diet log 322 (for example, recording particular foods or food groups, or foods not normally consumed by the user); and a medical condition 320 (for example, a recent or short-term medical condition or current unusual symptom). In some examples the user need not provide anything other than a stool for image capture of the stool by the optical sensor of the toilet apparatus.

In analysing the captured optical information of the user's stool 302, the machine learning analyser 310 may identify a value of a characterising parameter of the stool in the captured optical information 302. It may associate the value of the characterising parameter with a machine learnt lifestyle outcome; and may generate the personalised lifestyle recommendation for provision to the user based on the associated value of the characterising parameter. Examples of characterising parameters include: stool volume; stool colour profile; the presence of one or more predetermined colours; stool surface texture; stool shape; and stool fragmentation.

For example, the machine learning algorithm may quantify a parameter of the user's stool (e.g. the presence of the colour red in the captured optical information). A colour red (the characterising parameter) may indicate blood in the stool and thus may indicate, as determined in the training phase from members who also provided stools having the colour red present, constipation or a bowel disorder (examples of machine learnt lifestyle outcomes). Therefore, the machine learning algorithm 310 may associate the colour red with the lifestyle outcome of constipation or bowel disorder, and generate the personalised lifestyle recommendations to the user of "drink more water", "eat more dietary fibre" and "visit your doctor explaining the symptoms of red stool colouration". However, using analysis of stools by quantifying/qualifying certain appearance parameters is not essential to a machine learning process of evaluating captured optical information of stools to provide a lifestyle recommendation.

In some examples, the machine learning algorithm may be explicitly provided with medical knowledge from literature and medical experts to associate certain stool appearances with associated medical conditions. This "bootstrapping" process may use quantified parameters of stools to correlate certain stool characteristics with associated medical conditions.

The personalised lifestyle recommendation may be provided to the user 300 by the machine learning analyser 310 in some examples. The provision of the personalised lifestyle recommendation may be made directly to a device of the user, such as a portable electronic device a mobile phone, a tablet computer, a desktop computer, a laptop computer, a server, a wearable device, a wristband device, a fitness tracker device, a smartwatch, a user device or via a third-party device.

The machine learnt lifestyle outcomes which are determined in the training phase may indicate different information for the user. They may indicate: a characteristic of the user's stool; a diagnosis of a medical condition; an activity recommendation, and/or a dietary recommendation. The personalised lifestyle recommendation which is provided to the end user in the use phase of the machine learning analyser may also provide one or more of these indications.

A characteristic of the user's stool may comprise one or more of: stool volume; stool colour profile (e.g. the overall colour palette); the presence of one or more predetermined colours (e.g. dark red, white); stool surface texture (e.g. smooth, cracked, fluffy); stool shape (e.g. thin, fat); and stool fragmentation (e.g. several small pieces, one single piece, liquid).

An activity recommendation may comprise one or more physical activities for the user to avoid, and/or one or more physical activities for the user to perform (e.g. do some exercise in the morning before work, walk to work rather than drive, try and get an additional hour's sleep per night, attend a stress relief class once a week such as yoga, or avoid exercising after 8pm).

A dietary recommendation may comprise one or more foods or food groups the user should avoid (e.g. eat less dairy, stop eating high-gluten bread, limit your alcohol intake), one or more foods or food groups the user should consume (increase your vegetable portion intake, drink more water, try adding pulses to your diet), a recipe idea comprising one or more foods the user should consume (e.g. a vegetable lasagne recipe may be recommended to replace a user's meat lasagne food choice to help increase vegetable intake), and/or a recipe idea omitting one or more foods the user should avoid (e.g. try stir fry rice rather than stir fry egg noodles to lower gluten intake).

Use of toilet apparatus and machine learning analysers described here may benefit users who wish to improve their diet but have no pre-known underlying medial conditions requiring specific treatment. For example, the machine learning analyser may determine that the user has a high fat intake and that they may benefit from a lower fat intake even if the user is not considered obese or have any symptoms of disorders caused by a high-fat diet. The user may be determined to be suffering from stress and be recommended to sleep longer, take more breaks at work, or drink less alcohol to lower their stress levels. Use of toilet apparatus and machine learning analysers described here may also benefit people suffering from food intolerances and more severe conditions such as Crohn's and Coeliac's diseases. Diagnosing accurately which foods cause problems, and how badly the patient is affected by each, may greatly help the patient to manage the condition in daily life without requiring the user to (or allowing for a reduced need to), for example, manually keep a regular food diary or inspect their stools manually.

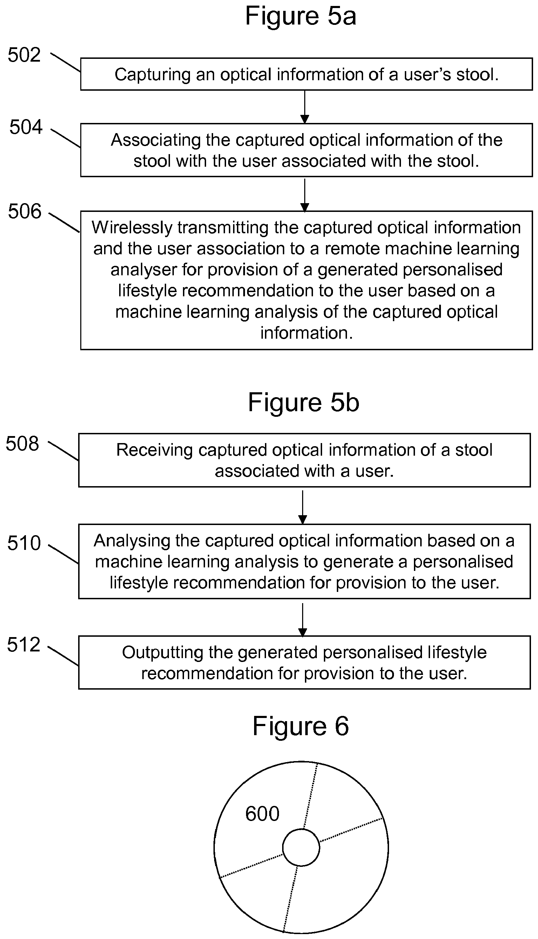

Figure 5a shows the main steps of a method of using the present apparatus. The method may be carried out at a toilet apparatus as described herein. The method comprises capturing optical information of a user's stool 502; associating the captured optical information of the stool with the user associated with the stool 504; and transmitting the captured optical information and the user association to a remote machine learning analyser for provision of a generated personalised lifestyle recommendation to the user based on a machine learning analysis of the captured optical information 506.

Figure 5b shows the main steps of a method of using the present apparatus. The method may be carried out at a machine learning analyser as described herein. The method comprises receiving captured optical information of a stool associated with a user 508; analysing the captured optical information based on a machine learning analysis to generate a personalised lifestyle recommendation for provision to the user 510; and outputting the generated personalised lifestyle recommendation for provision to the user 512.

Figure 6 shows an example computer-readable medium comprising a computer program configured to perform, control or enable the method of Figure 5a, 5b or any method described herein. The computer program may comprise computer code configured to perform the method(s). In this example, the computer/processor readable medium 600 is a disc such as a digital versatile disc (DVD) or a compact disc (CD). In other examples, the computer/processor readable medium 600 may be any medium that has been programmed in such a way as to carry out an inventive function. The computer/processor readable medium 600 may be a removable memory device such as a memory stick or memory card (SD, mini SD, micro SD or nano SD card).

It will be appreciated to the skilled reader that any mentioned apparatus/device and/or other features of particular mentioned apparatus/device may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/functional units.

In some examples, a particular mentioned apparatus/device may be pre-programmed with the appropriate software to carry out desired operations, and wherein the appropriate software can be enabled for use by a user downloading a "key", for example, to unlock/enable the software and its associated functionality. Advantages associated with such examples can include a reduced requirement to download data when further functionality is required for a device, and this can be useful in examples where a device is perceived to have sufficient capacity to store such pre-programmed software for functionality that may not be enabled by a user.

It will be appreciated that any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

It will be appreciated that any "computer" described herein can comprise a collection of one or more individual processors/processing elements that may or may not be located on the same circuit board, or the same region/position of a circuit board or even the same device. In some examples one or more of any mentioned processors may be distributed over a plurality of devices. The same or different processor/processing elements may perform one or more functions described herein.

It will be appreciated that the term "signalling" may refer to one or more signals transmitted as a series of transmitted and/or received signals. The series of signals may comprise one, two, three, four or even more individual signal components or distinct signals to make up said signalling. Some or all of these individual signals may be transmitted/received simultaneously, in sequence, and/or such that they temporally overlap one another.

With reference to any discussion of any mentioned computer and/or processor and memory (e.g. including ROM, CD-ROM etc.), these may comprise a computer processor, Application Specific Integrated Circuit (ASIC), field-programmable gate array (FPGA), and/or other hardware components that have been programmed in such a way to carry out the inventive function.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed examples may consist of any such individual feature or combination of features. In view of the foregoing description it will be evident to a person skilled in the art that various modifications may be made within the scope of the disclosure.

While there have been shown and described and pointed out fundamental novel features as applied to different examples thereof, it will be understood that various omissions and substitutions and changes in the form and details of the devices and methods described may be made by those skilled in the art without departing from the spirit of the invention. For example, it is expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or example may be incorporated in any other disclosed or described or suggested form or example as a general matter of design choice. Furthermore, in the claims means-plus-function clauses are intended to cover the structures described herein as performing the recited function and not only structural equivalents, but also equivalent structures. Thus, although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. A toilet apparatus comprising:
an optical sensor configured to capture optical information of a user's stool within a toilet bowl; and
a transmitter configured to transmit the captured optical information of a user's stool to a remote machine learning analyser;
wherein the toilet apparatus is configured to:
capture optical information of a user's stool using the optical sensor;
associate the captured optical information of the stool with the user associated with the stool; and
transmit, using the transmitter, the captured optical information and the user association to the remote machine learning analyser for provision of a generated personalised lifestyle recommendation to the user based on a machine learning analysis of the captured optical information.

2. The toilet apparatus of claim 1, wherein the toilet apparatus is one of: a toilet bowl, a toilet seat, an attachable module for a toilet bowl and an attachable module for a toilet seat.

3. The toilet apparatus of any preceding claim, wherein the optical sensor is configured to capture one or more of: a standard camera image, a stereoscopic image, a hyperspectral image, and a spectrometer measurement.

4. The toilet apparatus of any preceding claim, further comprising an output user interface configured to provide the personalised lifestyle recommendation to the user.

5. A machine learning analyser configured to:
receive captured optical information of a stool associated with a user; and
use machine learning analysis to generate a personalised lifestyle recommendation for provision to the user based on the captured optical information.

6. The machine learning analyser of claim 5, wherein the machine learning analyser is configured to:
use the machine learning analysis that is trained by associating respective lifestyle outcomes associated with further optical information of stools, wherein the further optical information of stools is from members of a population class linked to the user; and
based on the trained machine learning analysis, associate the captured optical information of the user's stool with one or more of the respective associated machine learnt lifestyle outcomes.

7. The machine learning analyser of claim 6, wherein the machine learning analyser is configured to generate the personalised lifestyle based on the respective associated machine learnt lifestyle outcomes associated with the captured optical information of the user's stool.

8. The machine learning analyser of any of claims 6-7, wherein:
the further optical information of stools from members of the population class linked to the user comprises previously captured optical information of stools associated with the user.

9. The machine learning analyser of any of claims 6-8, wherein the machine learnt lifestyle outcomes for the members of the population class linked to the user are based on one or more captured physiological indicators, the captured physiological indicators indicating one or more of: heart rate, breathing rate, blood pressure, weight, BMI, body temperature, body hydration, body perspiration, stress levels, glucose levels, blood oxygenation; and sleep activity.

10. The machine learning analyser of any of claims 6-9, further configured to:
identify a value of a characterising parameter of the captured optical information of the stool associated with the user;
associate the value of the characterising parameter with a machine learnt lifestyle outcome; and
generate the personalised lifestyle recommendation for provision to the user based on the associated value of the characterising parameter.

11. The machine learning analyser of any of claims 6-10, wherein the lifestyle outcomes indicate one or more of:
a characteristic of the user's stool;
a diagnosis of a medical condition;
an activity recommendation; and
a dietary recommendation.

12. The machine learning analyser of any of claims 5-11, further configured to provide the generated personalised lifestyle recommendation, by transmission to a user output device, of one or more of:
a visual message to display the generated personalised lifestyle recommendation,
an audio message to provide audio output indicating the generated personalised lifestyle recommendation, and
a haptic feedback indication to prompt the user that a personalised lifestyle recommendation has been generated.

13. A computer-implemented method comprising one or more of:
the method steps of:
capturing a optical information of a user's stool;
associating the captured optical information of the stool with the user associated with the stool; and
transmitting the captured optical information and the user association to a remote machine learning analyser for provision of a generated personalised lifestyle recommendation to the user based on a machine learning analysis of the captured optical information;
and the method steps of:
receiving captured optical information of a stool associated with a user;
analysing the captured optical information based on a machine learning analysis to generate a personalised lifestyle recommendation for provision to the user; and
outputting the generated personalised lifestyle recommendation for provision to the user.

14. A system comprising a toilet apparatus and a machine learning analyser;
the toilet apparatus comprising:
an optical sensor configured to capture optical information of a user's stool within a toilet bowl; and
a transmitter configured to transmit the captured optical information of a user's stool to a remote machine learning analyser;
wherein the toilet apparatus is configured to:
capture optical information of a user's stool using the optical sensor;
associate the captured optical information of the stool with the user associated with the stool; and
transmit, using the transmitter, the captured optical information and
the user association to the remote machine learning analyser; and
the machine learning analyser configured to:
receive the captured optical information of the stool and the user association; and
analyse the captured optical information based on a machine learning analysis to generate a personalised lifestyle recommendation for provision to the user.

15. A computer readable medium comprising computer program code stored thereon, the computer readable medium and computer program code being configured to, when run on at least one processor, perform one or more of:
the method of:
capturing a optical information of a user's stool;
associating the captured optical information of the stool with the user associated with the stool; and
transmitting the captured optical information and the user association to a remote machine learning analyser for provision of a generated personalised lifestyle recommendation to the user based on a machine learning analysis of the captured optical information
and the method of:
receiving captured optical information of a stool associated with a user;
analysing the captured optical information based on a machine learning analysis to generate a personalised lifestyle recommendation for provision to the user; and
outputting the generated personalised lifestyle recommendation for provision to the user.
